# EUROPEAN PATENT SPECIFICATION

(11) **EP 4 532 842 B1**
(45) Date of publication and mention of the grant of the patent: **01.07.2026**
(21) Application number: 23726196.1
(22) Date of filing: 23.05.2023
(51) Int. Cl.: E02D 1/02, G01K 7/00, G01N 33/24

(54) **PENETROMETER**
PENETROMETER
PENETROMETRE

(30) Priority: 01.06.2022 NL 2032052
(43) Date of publication of application: 09.04.2025
(73) Proprietor: GO-BEHEER B.V., 3117 RB Schiedam (NL)
(72) Inventor: SCHOON, Coen Johan, 9356 BZ Tolbert (NL); DE VRIES, Gerrit Tjerk, 3117 RB Schiedam (NL); USBECK, Regina, 27568 Bremerhaven (DE); KÄSSBOHRER, Johannes, 27568 Bremerhaven (DE)
(74) Representative: van Breda, Jacobus
(86) International application number: PCT/NL2023/050290
(87) International publication number: WO 2023/234772

(56) References cited:
- WO-A1-2018/074928
- WO-A1-2020/217113
- US-B2- 10 190 949

## Description

The invention relates to a cone penetrometer test (CPT) tube with a forward pointing cone equipped to be pushed into a soil at a controlled rate.

Wikipedia mentions that such a cone penetrometer test (CPT) tube is developed in the fifties of the previous century at the Dutch Laboratory for Soil Mechanics in Delft to investigate soft soils. Based on this history it has also been called the "Dutch cone test". Today, the CPT is one of the most used and accepted tools for soil investigation worldwide.

There is a need to complete the measurements that are possible with a cone penetrometer test (CPT) tube, with other measurements, in particular thermal measurements at relatively low depth of 0.3 m up to 10 m. Thermal measurements in connection with geology measurements are in general useful in oil and gas exploration, the offshore wind energy market, and in connection with gas hydrates, but such measurements are usually conducted at relatively larger depths and at great sea depth. In the known systems that are applied for those purposes, sensor-strings with a plurality of temperature sensors are vibrated into the ground.

It is an object of the invention to provide a method or system which is suitable for executing both a conventional cone penetrometer test measurement of a soil as well as a measurement of the thermal properties or conductivity of such soil. This is in particular desirable for assessment of a soil's suitability for cable burial onshore or in coastal zones with a sandy or clay sediment. The latter option in particular applies to offshore electric power cable burial and pipeline burial.

WO 2018/074928 discloses a probe arranged for subsurface penetration of a soil for measuring properties of the soil. The probe comprising a probe body and at least one fiber optical sensor. The at least one fiber optical sensor is arranged for measuring at least one of a cone resistance, sleeve friction, pore water pressure or inclination.

According to the invention a cone penetrometer test (CPT) tube is proposed with features according to one or more of the appended claims.

The cone penetrometer test (CPT) tube of the invention is provided with the feature that at a fixed distance outside and adjacent to the tube a longitudinally extending measurement probe is provided which is equipped for measuring thermal properties of the soil in which the tube is pushed, wherein the longitudinally extending measurement probe is fixed to the tube with two or more outriggers so as to ensure that the measurement probe can be pushed into the soil together with the tube while the tube and the probe maintain their relative position with reference to each other, and that the longitudinally extending measurement probe is provided with a heat supply portion and/or a cooling portion.

The heat supply portion and the cooling portion can be either passive or active. In the passive mode friction between the tube and probe and the soil can be used for heating. This can also be done active with dedicated heating elements. Active cooling is of course also possible.

Preferably the longitudinally extending measurement probe extends along at least 1 m. of the tube. With such a measurement instrument it is possible to simultaneously measure different parameters of the soil to be investigated.

Notable advantages of the combined tube and probe of the invention are the relatively quick mobilization and demobilization, the fact that deployment from small vessels is possible, and that the instrument requires minimal operator involvement enabling rapid and accurate testing. Further sediment disturbance is minimal which also contributes to accurate thermal property measurements. Because of the combined testing of the thermal properties with the conventional cone penetrometer test, measurement time is reduced by a factor two or more.

It is preferred that the tube and probe are mounted in a driving frame which is further provided with at least one centralizer and guide block for the tube and probe so as to be able to push the tube and probe in a controlled fashion into the soil. The driving frame is for instance provided with a set of driving rolls for driving the tube and probe, although other feasible driving options exist as well, such as driving chains driven by hydraulic engines. Another possible driving mode is for instance a conventional set of CPT rams as used onshore.

The invention will hereinafter be further elucidated with reference to the drawing of an exemplary embodiment of a combined cone penetrometer test (CPT) tube and thermal measurement probe according to the invention that is not limiting as to the appended claims.

In the drawing:
- figure 1 shows an instrument of the invention in a side view;
- figures 2A and 2B show the instrument of the invention in two different side views prior to entering into a soil to be investigated;
- figures 3A and 3B show the instrument of the invention in two different side views after entering into the soil to be investigated; and
- figure 4 shows a detail of the instrument with a centralizer and guide block for the tube and probe.

Whenever in the figures the same reference numerals are applied, these numerals refer to the same parts.

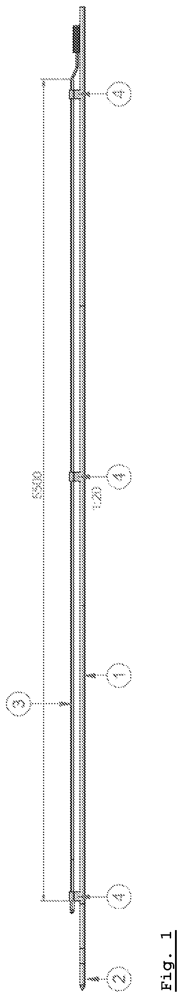

With particular reference to figure 1 a cone penetrometer test (CPT) tube 1 is shown with a forward pointing cone 2 equipped to be pushed into a soil at a controlled rate, wherein at a fixed distance outside and adjacent to the tube 1 a longitudinally extending measurement probe 3 is provided which is equipped for measuring thermal properties of the soil in which the tube 1 is to be pushed. It is clear from figure 1 that the longitudinally extending measurement probe 3 extends along more than 90% of the tube 1.

Figure 1 further shows that the longitudinally extending measurement probe 3 is fixed to the tube 1 with two or more outriggers 4 so as to ensure that the measurement probe 3 can be pushed into the soil together with the tube 1 while the tube 1 and the probe 3 maintain their relative position with reference to each other.

The longitudinally extending measurement probe 3 is provided with a heat supply portion and/or a cooling portion. The way this can be implemented is known to the skilled person and is therefore not shown. Examples are that the heat supply portion and the cooling portion can be either passive or active. In the passive mode friction between the tube 1 and probe 3 and the soil can be used for heating. This can also be done active with dedicated heating elements. Active cooling is of course also possible.

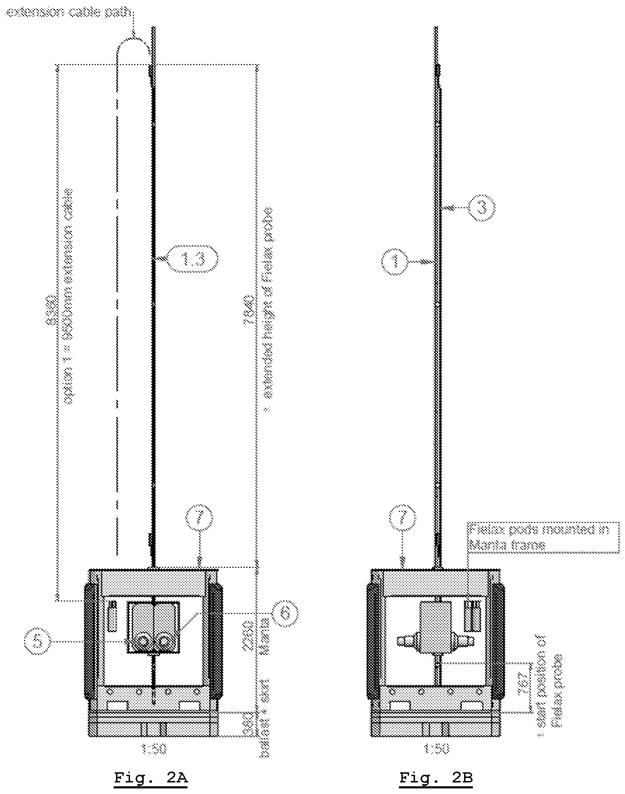

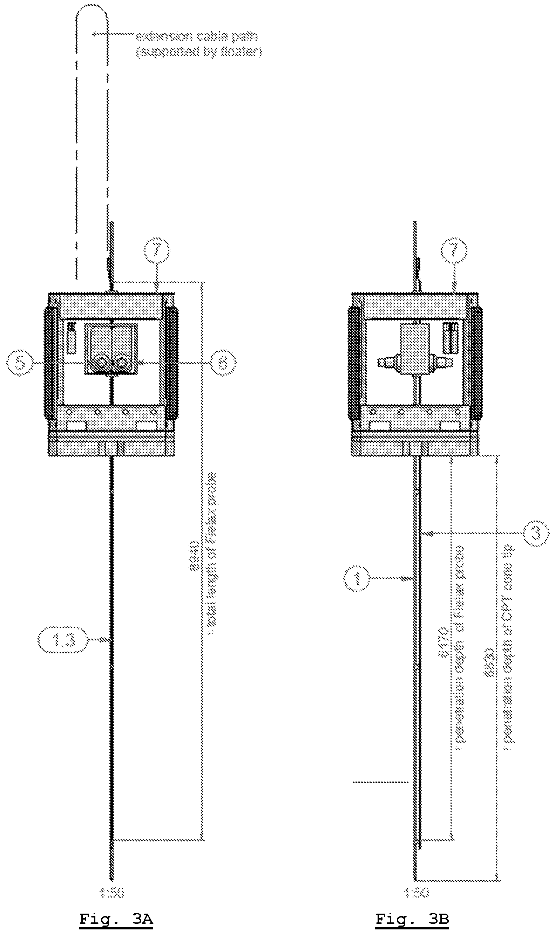

Figures 2A and 2B show the instrument of the invention in two different side views prior to entering into a soil to be investigated, whereas figures 3A and 3B show the instrument of the invention in two different side views after entering into the soil to be investigated. To enable pushing in or pulling out of the combined tube 1 and probe 3, the tube 1 and probe 3 are mounted in a driving frame 7. The figures show hydraulic engines 5, 6 that are arranged to drive a driving chain with clamping blocks to drive the tube 1 and probe 3. Other driving options are of course feasible also, such as a piston drive, hydraulic rams etc.

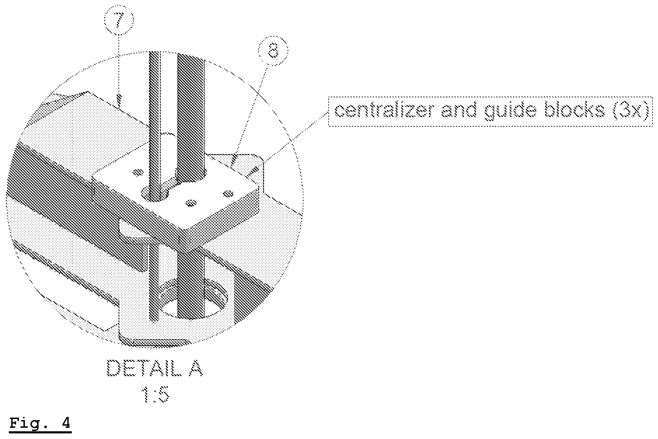

Figure 4 is a detail view showing that the frame 7 is further provided with at least one centralizer and guide block 8 for the tube 1 and probe 3.

## Claims

1. A cone penetrometer test (CPT) tube (1) with a forward pointing cone (2) equipped to be pushed into a soil at a controlled rate, wherein at a fixed distance outside and adjacent to the tube (1) a longitudinally extending measurement probe (3) is provided which is equipped for measuring thermal properties of the soil in which the tube (1) is pushed, wherein the longitudinally extending measurement probe (3) is fixed to the tube (1) with two or more outriggers (4) so as to ensure that the measurement probe (3) can be pushed into the soil together with the tube (1) while the tube (1) and the probe (3) maintain their relative position with reference to each other, **characterized in that** the longitudinally extending measurement probe (3) is provided with a heat supply portion and/or a cooling portion.

2. The cone penetrometer test (CPT) tube of claim 1, **characterized in that** the longitudinally extending measurement probe (3) extends along at least 1 m. of the tube (1).

3. The cone penetrometer test (CPT) tube of any one of claims 1-2, **characterized in that** the tube (1) and probe (3) are mounted in a driving frame (7) which is further provided with at least one centralizer and guide block (8) for the tube (1) and probe (3).

4. The cone penetrometer test (CPT) tube of claim 3, **characterized in that** the driving frame (7) is provided with a set of driving rolls (5, 6).

## Patentansprüche

1. Gestänge (1) für eine Drucksondierung (CPT) mit einem nach vorne gerichteten Kegel (2), der dazu ausgestattet ist, mit einer gesteuerten Geschwindigkeit in einen Boden geschoben zu werden, wobei in einem festen Abstand außerhalb und benachbart zu dem Gestänge (1) eine sich in Längsrichtung erstreckende Messsonde (3) vorgesehen ist, die dazu ausgestattet ist, thermische Eigenschaften des Bodens zu messen, in den das Gestänge (1) geschoben wird, wobei die sich in Längsrichtung erstreckende Messsonde (3) an dem Gestänge (1) mit zwei oder mehr Auslegern (4) befestigt ist, um sicherzustellen, dass die Messsonde (3) zusammen mit dem Gestänge (1) in den Boden geschoben werden kann, während das Gestänge (1) und die Messsonde (3) ihre relative Position in Bezug aufeinander beibehalten, **dadurch gekennzeichnet, dass** die sich in Längsrichtung erstreckende Messsonde (3) mit einem Wärmezufuhrabschnitt und/oder einem Kühlabschnitt versehen ist.

2. Gestänge (CPT) für eine Drucksondierung nach Anspruch 1, **dadurch gekennzeichnet, dass** die sich in Längsrichtung erstreckende Messsonde (3) sich über mindestens 1 m des Gestänges (1) erstreckt.

3. Gestänge für eine Drucksondierung (CPT) nach einem der Ansprüche 1 bis 2, **dadurch gekennzeichnet, dass** das Gestänge (1) und die Messsonde (3) in einem Antriebsrahmen (7) angeordnet sind, der ferner mit mindestens einem Zentrier- und Führungsblock (8) für das Gestänge (1) und die Messsonde (3) versehen ist.

4. Gestänge für eine Drucksondierung (CPT) nach Anspruch 3, **dadurch gekennzeichnet, dass** der Antriebsrahmen (7) mit einem Satz von Antriebsrollen (5, 6) versehen ist.

## Revendications

1. Tube de pénétromètre statique (CPT) (1) muni d'un cône (2) orienté vers l'avant et configuré pour être poussé dans un sol à une vitesse contrôlée, dans lequel, à une distance fixe à l'extérieur et adjacente au tube (1), est disposée une sonde de mesure (3) s'étendant longitudinalement et configurée pour mesurer des propriétés thermiques du sol dans lequel le tube (1) est poussé, la sonde de mesure (3) s'étendant longitudinalement étant fixée au tube (1) au moyen d'au moins deux bras de liaison (4) de manière à garantir que la sonde de mesure (3) peut être poussée dans le sol conjointement avec le tube (1) tout en maintenant leur position relative l'un par rapport à l'autre, **caractérisé en ce que** la sonde de mesure (3) s'étendant longitudinalement est pourvue d'une portion de chauffage et/ou d'une portion de refroidissement.

2. Tube de pénétromètre statique (CPT) selon la revendication 1, **caractérisé en ce que** la sonde de mesure (3) s'étendant longitudinalement s'étend sur au moins 1 m du tube (1).

3. Tube de pénétromètre statique (CPT) selon l'une quelconque des revendications 1 à 2, **caractérisé en ce que** le tube (1) et la sonde (3) sont montés dans un bâti d'entraînement (7) qui est en outre pourvu d'au moins un dispositif de centrage et de guidage (8) pour le tube (1) et la sonde (3).

4. Tube de pénétromètre statique (CPT) selon la revendication 3, **caractérisé en ce que** le bâti d'entraînement (7) est pourvu d'un ensemble de rouleaux d'entraînement (5, 6).
